# EUROPEAN PATENT APPLICATION

(11) **EP 4 782 491 A1**
(43) Date of publication of application: **29.07.2026**
(21) Application number: 25154471.4
(22) Date of filing: 28.01.2025
(51) Int. Cl.: C08J 11/16, C07C 4/22

(54) **METHOD FOR DEPOLYMERIZING VINYL-BASED AROMATIC OR HETEROAROMATIC POLYMERS OR COPOLYMERS**

(71) Applicant: ETH Zurich, 8092 Zurich (CH)
(72) Inventor: FEDOROV, Alexey, 8180 Bülach (CH); TIAN, Puyang, 8052 Zürich (CH)

(57) **Abstract**

The present invention relates to a method for depolymerizing vinyl-based aromatic or heteroaromatic polymers or copolymers in the absence of a heterogenous nickel catalyst, including the steps of
providing a strong electron donor and a vinyl-based aromatic or heteroaromatic polymer or copolymer,
reacting the vinyl-based aromatic or heteroaromatic polymer or copolymer with the strong electron donor in the presence of H₂ at a temperature between 130°C and 275°C to obtain platform chemicals.

## Description

The present invention relates to a method for depolymerizing vinyl-based aromatic or heteroaromatic polymers or copolymers, in particular styrenic polymers.

Styrenic polymers are a group of synthetic materials that comprise styrene monomers. They are known for their strength, rigidity, and versatility, making them suitable for numerous applications, including packaging materials, consumer goods, automotive components, and household items. Among them, polystyrene (PS) stands out, with tens of millions of tons produced annually. However, the widespread use of styrenic polymers raises challenges related to recycling and environmental sustainability.

The shift from a linear to a circular economy is imperative from both ecological and economic perspectives, driven by concerns over climate change, environmental pollution, population growth, and resource dependency. To align with the principles of a circular economy, it is essential to transform polymer waste, such as styrenic polymers, into valuable chemicals. Despite this necessity, efficiently recycling and repurposing polystyrene remains a challenge due to various factors. One promising method for upgrading polystyrene is net hydrogenolysis, which involves replacing carbon-carbon bonds with carbon-hydrogen bonds under a hydrogen atmosphere. While significant progress has been made in the hydrogenolysis of polyethylene and polypropylene, advancements in polystyrene hydrogenolysis have been slower.

For instance, uncatalyzed hydrogenolysis can produce a mixture of styrene and ethylbenzene but requires high temperatures of about 480°C (J. Am. Chem. Soc. 2023, 145, 16218-16227). A combined approach of uncatalyzed pyrolysis under hydrogen and catalyzed hydrogenation of polystyrene into ethylbenzene using iron or cobalt catalysts has been explored, yet it faces limitations due to high temperatures and substantial catalyst requirements. Additionally, while hydrogen plasma techniques for non-thermal hydrogenolysis have been used, they primarily yield small hydrocarbons, mainly ethene.

The use of transition metal catalysts, such as nickel and ruthenium, enables the hydrogenolysis of polystyrene with a catalyst-controlled selectivity and at slightly lower temperatures relative to the uncatalyzed reaction. However, their use presents significant economic and environmental challenges. For example, using a nickel/silica catalyst, polystyrene can be converted at 300 °C and 70 bar of hydrogen pressure into lubricant oils, which include alkanes, alkylated aromatics, and styrene oligomers (ACS Catal. 2024, 14, 5389-5402; Cell Rep. Phys. Sci. 2021, 2, 100332). In contrast, a ruthenium-loaded zeolite predominantly produces methane at 350 °C and 50 bar of hydrogen pressure (Cell Rep. Phys. Sci. 2021, 2, 100332). When hydrogen is sourced from methanol, the hydrogenolysis of polystyrene can take place at 280 °C with a ruthenium/silica catalyst, generating alkylbenzenes, diphenyl alkanes, and polyaromatic compounds. Furthermore, a ruthenium catalyst on niobium oxide selectively cleaves specific carbon bonds in polystyrene around 300 °C at 5 bar of hydrogen, achieving 65% selectivity for benzene, though it requires an equal mass of catalyst and polystyrene for optimal results. Another catalyst, NbOPO₄, demonstrates a 53% selectivity for benzene under similar conditions (Angew. Chem. Int. Ed. 2021, 60, 5527-5535).

Xu et al., ChemSusChem 2024, 17, e202400474, report on Hydrogen Atom Transfer (HAT) for polystyrene (PS) upcycling, enabling the conversion of waste polystyrene into valuable products like benzoic acid. Using FeCl₂ under 400-nm light, 100% conversion of polystyrene dimers was achieved with about 90% selectivity, though polystyrene required 66 hours for a 60% yield. Subsequent studies with FeCl₃ and other catalysts produced diverse benzyl compounds with moderate selectivity for benzoic acid.

Thus, while various methods exist for converting polystyrene into useful chemicals, a sustainable, selective, low-temperature, and high-yielding process for upcycling styrenic polymers into valuable platform chemicals, such as alkylated aromatics, has yet to be fully realized.

The problem of the present invention is therefore to develop an efficient and eco-friendly process for the upcycling of vinyl-based aromatic or heteroaromatic polymers or copolymers, in particular styrenic polymers into valuable platform chemicals.

The problem is solved by the method according to the present invention. Further preferred embodiments are subject of dependent claims 2 to 15.

With the method according to the present invention it is possible to depolymerize vinyl-based aromatic or heteroaromatic polymers or copolymers, and in particular styrenic polymers, into platform chemicals in a very efficient manner. The method is conducted in the absence of a heterogenous nickel catalyst and includes the steps of
a) providing a strong electron donor and a vinyl-based aromatic or heteroaromatic polymer or copolymer,
b) reacting the vinyl-based aromatic or heteroaromatic polymer or copolymer with the strong electron donor in the presence of H₂ at a temperature between 130°C and 275°C to obtain platform chemicals.

Thanks to the method according to the present invention, vinyl-based aromatic or heteroaromatic polymers or copolymers, and in particular styrenic polymers can be depolymerized into platform chemicals with unprecedented efficiency and sustainability. The method according to the present invention employs inexpensive and readily available reagents, substantially lowering the cost of the depolymerization process compared to conventional approaches. Furthermore, it minimizes waste, promotes a circular economy, and enhances environmental sustainability while preserving essential resources.

Moreover, the reaction conditions employed in this process are mild, making it safe and cost-efficient. These gentle conditions reduce the formation of unwanted by-products, enhancing the overall purity of the final products. Most importantly, the method exhibits superior selectivity towards valuable platform chemicals, in particular benzene, toluene, ethylbenzene, and cumene. Thus, this high selectivity maximizes the yield of desirable compounds. Ethylbenzene is a particularly favored platform chemical due to its crucial role as an intermediate in the production of styrenic polymers. Consequently, this presents a compelling advantage over traditional methods that often yield a mixture of products, complicating downstream processing and reducing overall economic viability. Additionally, the method presented in this invention provides enhanced thermal stability and significantly reduces the likelihood of unwanted re-polymerization reactions in styrene, presenting a substantial improvement over the reaction mixtures found in prior art. Thus, the method according to the present invention allows to convert polystyrene to the highly desirable platform chemical ethylbenzene under milder conditions and with a higher selectivity compared to the reported transition metal catalysts:

| Catalyst | P in bar | T in °C | T in hours | Product | Selectivity in % | Yield in % |
|---|---|---|---|---|---|---|
| Ni/SiO₂ | 30 | 300 | 2 | Cycloalkanes, aromatics, polystyrene oligomers, and C₁₀₀₊ | | More than 99 |
| Ni/SiO₂ | 70 | 300 | 6 | Alkanes, alkyl aromatics, polystyrene oligomers | | 70 |
| Ru/zeolite | 50 | 350 | 4 | CH₄, C₂H₆ and C₃H₈ | 92 (CH₄) | More than 99 |
| NbOPO₄ | 5 | 280 | 12 | Benzene, alkylbenzenes and bicyclic compounds | 53 (benzene) | 52 |
| Ru/Nb₂O₅ | 5 | 300 | 16 | Benzene, alkylbenzenes and bicyclic compounds | 65 (benzene) | 76 |
| One embodime nt of the method according to the present invention | 10 | 220 | 10 | Benzene, toluene, ethyylbenzene and cumene | 79 (ethylbenzene ) | 72 |

As mentioned above, the reaction takes place in the absence of a heterogenous nickel catalyst. Nickel catalyst produce under H₂ at temperatures above 120-130°C Ni(0) nanoparticles, which is highly undesirable. It ensures an environmentally friendly process, eliminates reliance on nickel, and aligns with sustainable practices by reducing waste and resource consumption. Ideally, the reaction takes place without any transition metal catalysts, resulting in an efficient and very eco-friendly process.

The method according to the present invention is a formal hydrogenolysis of vinyl-based aromatic or heteroaromatic polymers or copolymers, and in particular of styrenic polymers, which takes place in the presence of hydrogen. Hydrogenolysis is a chemical process that involves the cleavage of carbon-to-carbon (C-C) bonds through the addition of hydrogen. In the presence of H₂, the strong electron donor helps to facilitate the formal hydrogenolysis reaction.

Without wishing to be bound to it, it is assumed that the mechanism could be as follows (illustrated with polystyrene as an example): It is proposed that hydrogen radicals (H·) are formed through the interaction of a strong electron donor (SED) with hydrogen (H₂) in the initiation step. The thus formed hydrogen radical abstracts an H· from the benzyl position of polystyrene, leading to a benzyl radical in the polymer chain and regenerating H₂. A β-scission process then sets in to depolymerize PS to styrene. A radical hydrogenation of styrene in the reaction conditions gives ethylbenzene (5). Adding benzyl radical to styrene provides a dimer radical that can further fragment via a β-scission process, yielding toluene and cumene. This mechanism accounts for the experimentally observed ethylbenzene, toluene, and cumene products main products, as well as for the presence of minor amounts of dimers.

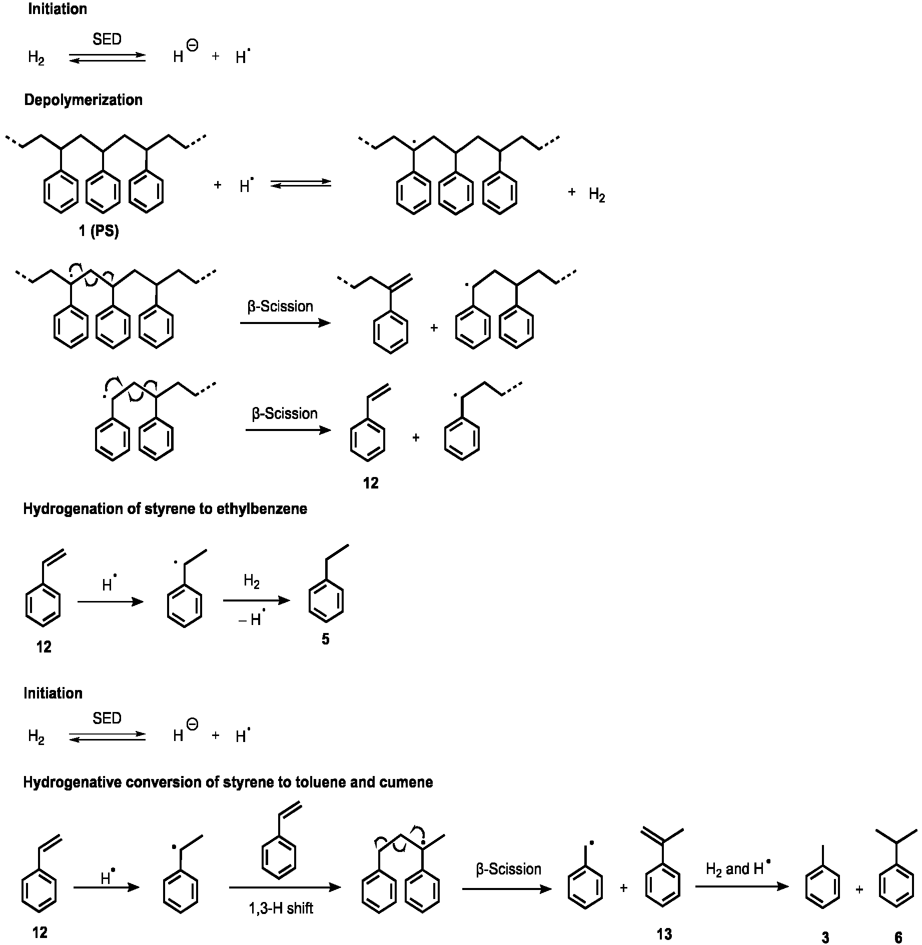

Within the context of the present invention, the term "vinyl-based aromatic or heteroaromatic polymers or copolymers" refers to polymers derived from vinyl monomers. Further, they contain aromatic or heteroaromatic structures in their side chains. A heteroaromatic structure contains one or more heteroatoms selected from the group consisting of nitrogen, oxygen, and sulfur, with nitrogen being the preferred heteroatom. The term "vinyl-based aromatic or heteroaromatic polymer or copolymer" encompasses styrenic polymers as defined below, as well as polymers such as poly(4-vinylpyridine).

Within the context of the present invention, the term "styrenic polymer" stands for a type of polymer that is derived from styrene monomers or combinations of styrene monomers with other monomers (i.e. copolymers), characterized by a backbone with aromatic rings attached as side chains. This definition encompasses both homopolymers, which consist solely of styrene units, and copolymers, which are formed by the polymerization of styrene with one or more different monomers. Additionally, this definition includes derivatives of styrene monomers, where substitutions or modifications occur on the aromatic ring (e.g., methyl or tert-butyl) or the α-carbon of the monomer. Typical examples of styrenic polymers under this definition include, but are not limited to, polystyrene, poly(a-methylstyrene), poly(p-methylstyrene), poly(vinyltoluene), poly(p-tert-butylstyrene), and their copolymers with monomers such as acrylonitrile and butadiene.

Within the context of the present invention, the term "depolymerizing" stands for the process of breaking down the vinyl-based aromatic or heteroaromatic polymer or copolymer into platform chemicals, in particular non-alkylated or alkylated aromatic or heteroaromatic compounds such as benzene, toluene, ethylbenzene, cumene, methylpyridine, ethylpyridine and isopropylpyridine.

In the context of the present invention, "platform chemicals" are key compounds, particularly non-alkylated or alkylated aromatic compounds, that serve as versatile building blocks for producing a wide range of value-added chemicals, materials, and industrial products. Alkylated aromatic compounds are chemical compounds formed by introducing one or more alkyl groups (such as methyl or ethyl) into an aromatic ring, such as toluene or ethylbenzene. Examples of alkylated heteroaromatic compounds are methylpyridine and ethylpyridine.

Within the context of the present invention, the term "a strong electron donor" refers to a chemical compound that has a high tendency to donate electrons in the presence of H₂. This process leads to the formation of a hydrogen radical formed via an interaction of a strong electron donor with H₂, which serves as a reactive intermediate and plays a crucial role in the subsequent reaction.

Preferably, the strong electron donor is selected from the group consisting of a metal hydride, an *in* situ generated alkali metal hydride, alkali naphtalide, in particular sodium naphtalide, alkali diaryl dianion salt, alkalides, and electrides. All of these compounds facilitate efficient electron transfer during the depolymerization reaction.

The term "metal hydride" encompasses both alkali metal hydrides, which are formed with alkali metals such as lithium (Li), sodium (Na), and potassium (K), as well as various hydrides from other metal categories. These include alkaline earth metal hydrides, such as calcium hydride (CaH₂) and magnesium hydride (MgH₂), and transition metal hydrides, including compounds like iron hydride (FeH₂). Additionally, metal hydrides can consist of boron and aluminum hydrides, such as sodium borohydride (NaBH₄) and lithium aluminum hydride (LiAlH₄). Alkali metal hydrides are favored for their efficiency and environmental benefits. The term "alkalide" refers to a chemical compound in which alkali metal atoms are anions, i.e. having a negative charge. The term "electride" refers to an ionic compound in which an electron serves the role of the anion.

In one embodiment of the present invention, the metal hydride, and preferably the alkali metal hydride, is used in the presence of a support. Said support is preferably selected from the group consisting of carbon, MgO, ZnO, CaO, silica, alumina, zeolites, and mesoporous silica, with carbon being the most preferred. It has been found that the presence of a support increases the efficiency of the reaction, likely because it allows for better dispersion and reduces the tendency to agglomerate. Furthermore, alkali metal hydrides on a support don't leach to organic solvents and can be recycled and reused.

Preferably, the strong electron donor is an alkali metal hydride, most preferably selected from the group consisting of potassium hydride, sodium hydride, and lithium hydride, all of which are easily accessible. The ability of these alkali metal hydrides to act as strong electron donors allows for milder reaction conditions, facilitating the depolymerization of vinyl-based aromatic or heteroaromatic polymers or copolymers without the need for excessive energy input. This energy efficiency is essential in green chemistry, which seeks to minimize energy consumption while maximizing product yields.

Especially good results can be achieved when using potassium hydride, particularly in its carbon-supported form. While the method is effective with potassium hydride, it has been found that potassium hydride on carbon significantly enhances depolymerization. In the case of polystyrene, it yields non-alkylated and alkylated platform chemicals, in particular benzene, toluene, ethylbenzene, and cumene. Notably, the highly desirable platform chemical ethylbenzene can be obtained with a selectivity of at least 79% through the depolymerization of polystyrene using potassium hydride on carbon. The efficiency of the method according to the present invention not only promotes the breakdown of vinyl-based aromatic or heteroaromatic polymers or copolymers but also allows for the recovery of valuable aromatic compounds that are crucial in various chemical industries.

Alternatively, the alkali metal hydride can be generated *in situ.* This *in situ* generation enhances safety and simplifies handling, especially for scale-up in industrial applications. Potassium hydride (KH) can be produced *in situ* from potassium alkoxides and silanes, preferably using KOtBu and Et₃SiH (Stoltz-Grubbs system). Alongside alkylated and non-alkylated monocyclic aromatics, styrene dimers can be formed during the depolymerization of styrenic polymers. Also these styrene dimers can serve as valuable platform chemicals for various applications. For instance, they act as intermediate products in the production of resins and specialty polymers, where they enhance properties such as strength and temperature resistance. Additionally, they are utilized as additives in plastics to achieve specific mechanical or thermal properties and as plasticizers to increase the flexibility of materials.

Alkali metal hydrides, in particular sodium hydride, potassium hydride, rubidium hydride, and cesium hydride, can also be generated *in situ* from their corresponding metal hydroxide MiOH and magnesium, wherein M₁ is selected from the group consisting of sodium, potassium, rubidium and cesium (Inorg. Chem., 2024, 63, 3047). The reactions occurred in an autoclave reactor with paraffin oil at 250 °C and 14 bar H₂ pressure. This method allows for particularly straightforward handling and is highly cost-effective due to the inexpensive materials used. Furthermore, the use of air-stable precursors like KOH not only enhances safety during storage and handling but also supports costeffectiveness due to the availability of these materials. Alkali metal hydrides can also be produced by heating an n-alkyl metal alkyl in an alkane solvent. For example, n-BuLi decomposes to LiH via betahydride elimination, resulting in the formation of 1-butene. A similar product distribution was observed in the depolymerization reaction of polystyrene using n-BuLi as a precursor, compared to KH/C under similar reaction conditions (220°C, 50 bar, 20 h, 10 mol%).

As mentioned before, the method according to the present invention takes place in the presence of H₂. Preferably, H₂ is added with a pressure of 1 to 50 bar, preferably 5 to 30 bar. With this pressure, an excellent depolymerization rate with high selectivity can be achieved.

Preferably, the reaction is conducted in an apolar solvent, even more preferably an alkane solvent, most preferably decane and dodecane, and ideally dodecane. The nonpolar nature of these solvents, especially dodecane, effectively dissolves the obtained platform chemicals. Dodecane's high boiling point ensures stability during the depolymerization reaction, even at elevated temperatures, while its inertness prevents interference with the strong electron donor. Additionally, dodecane helps regulate the viscosity of the reaction mixture, ensuring uniform heat distribution and efficient mixing. After the reaction, the platform chemicals can be easily separated, and dodecane can be readily removed and reused.

It is also possible to conduct the reaction in the melt of the vinyl-based aromatic or heteroaromatic polymers or copolymers, in particular styrenic polymers, preferably in the absence of a further solvent or in the presence of a small amount of solvent. This results in a reduction in the overall environmental footprint, as it minimizes the consumption of chemical resources, leading to a more sustainable and environmentally friendly process. In this case, the reaction is typically carried out just above the melting point of the polymer.

The concentration of the strong electron donor for polystyrene is preferably between 1 and 20 mol%, even more preferably between 5 and 10 mol%, and most preferably about 10 mol%. Experiments showed that using 1 mol % of the strong electron donor resulted in a notable consumption of H₂ during the reaction. These results indicate that low catalytic amounts of the strong electron donor are sufficient to induce the depolymerization-hydrogenation sequence of polystyrene. However, the best results could be obtained within the preferred range. For copolymers and vinyl heterocycle polymers, a higher concentration of the strong electron donor is advantageous. Particularly good results can be achieved with a molar excess of the strong electron donor, preferably around 1.25.

Preferably, the method according to the present invention is conducted at a temperature between 160°C and 250°C, more preferably between 160°C to 240°C, even more preferably between 160°C and 220°C, which is significantly lower than the reaction conditions used in the state of the art with transition metal catalysts. This very energy-efficient manner of depolymerizing styrenic polymers and producing valuable platform chemicals, such as non-alkylated and alkylated aromatics, is highly advantageous.

The reaction can occur over a duration of 1 to 12 hours. Even under such mentioned mild conditions, it is possible to selectively degrade the polymers with high yield within such a short period.

Depending on the nature of the strong electron donor, the reaction can be conducted in an inert atmosphere, for example, under nitrogen.

In one embodiment of the present invention the styrenic polymer is polystyrene, preferably selected from the group consisting of expanded polystyrene (EPS) and extruded polystyrene (XPS). The method of the present invention can be utilized to upcycle polystyrene found in various applications, such as packaging materials, the construction industry, consumer goods, medical technology, and plastics and coatings.

In another embodiment of the present invention, the styrenic polymer is a copolymer, preferably selected from the group consisting of styrene-butadiene rubber (SBR), acrylonitrile-butadiene-styrene (ABS), styrene-acrylonitrile (SAN), styrene-ethylene-butylene-styrene (SEBS), styrene-isoprene-styrene (SIS), styrene-maleic anhydride (SMA), poly(styrene-co-maleic anhydride), and styrene-ethylene/propylene-styrene (SEPS), preferably styrene-butadiene Rubber (SBR), acrylonitrile-butadiene-styrene (ABS), styrene-acrylonitrile (SAN), styrene-ethylene-butylene-styrene (SEBS), styrene-isoprene-styrene (SIS), and styrene-ethylene/propylene-styrene (SEPS). These polymers, particularly styrene-maleic anhydride (SMA) and poly(styrene-co-maleic anhydride), depolymerize significantly better in the presence of a high concentration of a strong electron donor, preferably in stoichiometric amounts or greater.

In a further embodiment of the present invention, the vinyl-based aromatic or heteroaromatic polymer is poly(4-vinylpyridine). Although it is slightly less selective than for styrenic polymers, the method according to the present invention allows for the degradation of poly(4-vinylpyridine) into extremely valuable platform chemicals such as methylpyridine, ethylpyridine, and isopropylpyridine.

The method of the present invention can be utilized to upcycle the copolymers found in various applications, including automotive components - particularly tires -consumer electronics, packaging materials, and construction materials.

### Figures

Figure *1* shows the optimization of the reaction conditions, a) Variation of temperature (140-220 °C) using 10 mol % KH/C loading, 10 bar H₂, and 10 h duration. b) Variation of reaction time (1-10 h) using 10 mol % KH/C loading, 220 °C, and 10 bar H₂. c) Variation of KH/C loading (1-10 mol %) using 220 °C, 10 bar H₂ and 10 h. d) Variation of H₂ pressure (2-10 bar) using 10 mol % KH/C loading, 220 °C, and 10 h.
Figure 2 shows the comparison of depolymerization of the reagent grade, package foam, and food-grade polystyrene using 10 mol % KH/C loading, 220 °C, 10 or 20 bar H₂ for 10 h.
Figure 3 shows recyclability tests of KH/C using 10 mol % KH/C loading, 220 °C, 30 bar H₂, and 10 h.

### Experiments

### KH/C synthesis protocol:

KH/C with a nominal KH loading of 30 wt % was prepared by a melt impregnation method. The carbon support (graphene nanoplatelets with a BET surface area of ca. 500 m² g⁻¹, Sigma Aldrich) was pre-treated under a N₂ flow (30 ml min⁻¹) at 550 °C (ramping rate was 10 °C min⁻¹) for 6 h and then stored in a N₂-filled glovebox. In the glovebox, 150 mg of metallic potassium slices (< 3 mg per slice) and 350 mg of the pre-treated carbon support were physically mixed and placed into an autoclave with a glass liner. The autoclave was sealed, brought out from the glovebox, and connected to a high-pressure H₂ line. The line was purged 5 times with 10 bar of H₂, the autoclave pressurized to 10 bar of H₂, heated to 110 °C (ramping rate was 5 °C min⁻¹) and held at this temperature for 10 h. After cooling down, the autoclave was purged with Ar and transferred into the N₂-filled glovebox. The solid material in the autoclave was collected, carefully mixed using mortar and pestle, and stored in a glovebox.

### Polystyrene (PS) and styrene-butadiene rubber (SBR) depolymerization protocol:

In a N₂-filled glovebox, 50 mg of KH/C was combined with a desired amount of polystyrene (reagent-grade granules, Sigma Aldrich, or consumer-grade packaging foam or cutlery, broken into pieces) or SBR (reagent-grade granules, Sigma Aldrich) and 10 ml of degassed dodecane solvent was added into an autoclave equipped with a glass liner. The autoclave was sealed, brought out from the glovebox, connected to a high-pressure H₂ line, purged 3 times to 100 bar H₂, pressurized to a set pressure of H₂, heated to a desired temperature (ramping rate was 5 °C min⁻¹) and held at this temperature for a desired time. Unless specified otherwise, the stirring rate was 500 rpm. After the reaction, the autoclave was opened in the air except for the recycling experiments, and the reaction mixture was filtered (filter paper). After adding a known amount of mesitylene internal standard, the obtained liquid was analyzed by gas chromatography with mass spectrometry and flame ionization detectors (GC-MS and GC-FID). For the recycling experiments, the autoclave was purged with Ar and transferred into a N₂-filled glovebox after a test. The supernatant of the reaction mixture was collected, and a new batch of polystyrene and dodecane was added for the subsequent test.

### The following equations were used to calculate product yield in the case of PS:

The yield of monocyclic compounds (%) = (molar amount of alkyl benzene)/(molar amount of PS) × 100%. The yield of bicyclic compounds (%) = 2 × (molar amount of a diphenyl alkane)/(molar amount of PS) × 100%.

### The following equations were used to calculate product yield in the case of SBR:

The yield of monocyclic compounds (%) = (molar amount of alkyl benzene)/(molar amount of styrene monomer in SBR) × 100%.

### Results

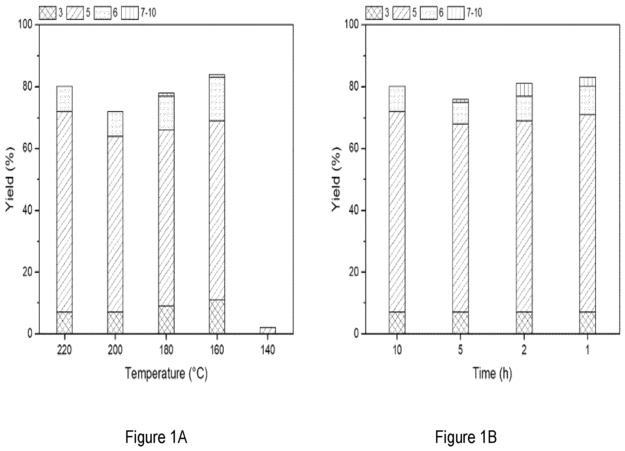

Combining polystyrene (3.75 mmol calculated based on the monomer's molecular weight, reagent grade polystyrene is denoted as **1**-PS_{reagent}, average Mw ca. 280,000 according to gel permeation chromatography, GPC) with 10 mol % KH/C under 10 bar H₂ at 220 °C for 10 h gives 7 % toluene **(3),** 65 % ethylbenzene **(5)** and 8 % cumene **(6)** (Figure 1a: Variation of temperature (140-220 °C) using 10 mol % KH/C loading, 10 bar H₂, and 10 h duration.). Maintaining 10 mol % KH/C loading, 10 bar H₂, and 10 h duration, the reaction temperature can be reduced to 160 °C with a similar combined yield of **3, 5,** and **6** of 83 %; however, decreasing the temperature further to 140 °C yields merely 2 % of **5** as the only product (Figure 1a). These results show that the depolymerization of polystyrene can proceed at temperatures as low as 160 °C.

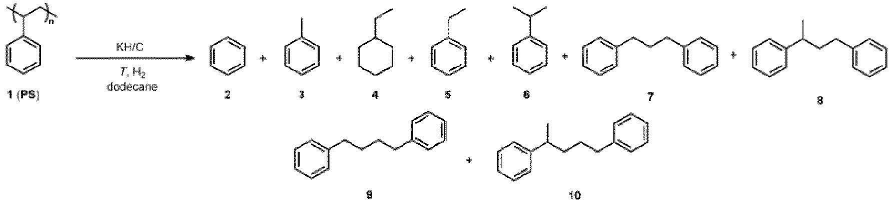

With a 10 mol% KH/C loading under 220 °C and 10 bar H₂, the depolymerization of polystyrene **(1)** can be performed in one hour without a notable change in the yield of monomers **3, 5,** and **6** relative to the 10 h experiment. Specifically, 80 % combined yields of **3, 5,** and **6** are obtained after 10 h and 1 h (Figure 1b: Variation of reaction time (1-10 h) using 10 mol % KH/C loading, 220 °C, and 10 bar H₂.). Therefore, the depolymerization reaction is facile in the mentioned conditions.

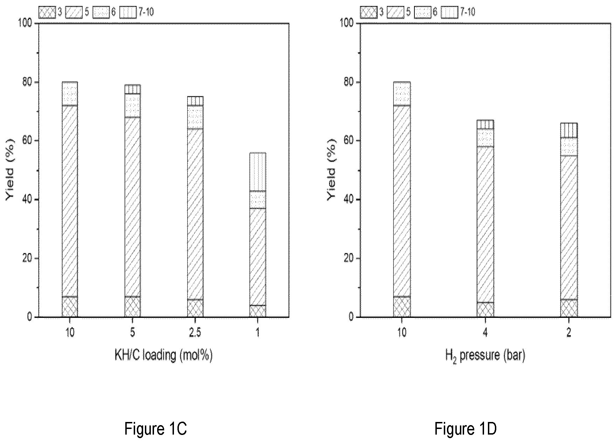

At 220 °C, 10 bar H₂, and using 10 h experiment duration, the KH/C loading can be reduced to 2.5 mol % with only a minor decrease of a combined yield of **3, 5,** and **6,** i.e., from 80 % with 10 mol % KH/C to 72 % with 2.5 mol % KH/C. Furthermore, the KH/C loading can be reduced to 1 mol %, although leading to a lower 43 % yield of **3, 5,** and **6** and an increased 13 % yield of diphenyl alkanes **7-10.** Note that experiments using 1 mol % KH/C loading resulted in a notable consumption of H₂ during the reaction, decreasing the H₂ pressure to ca. 1 bar at the end of the experiment. These results show that low catalytic amounts of KH are sufficient to induce the depolymerization-hydrogenation sequence of polystyrene (Figure 1c: Variation of KH/C loading (1-10 mol %) using 220 °C, 10 bar H₂ and 10 h.).

The results under 10 bar H₂ were compared to experiments using initial 4 bar and 2 bar H₂ to probe the pressure effect using 10 mol % KH/C loading, 220 °C, and 10 h reaction duration. The combined yield of **3, 5,** and **6** was 64 % and 61 % at 4 bar and 2 bar H₂, respectively, which is lower than 80 % under 10 bar H₂ (Figure 1d: Variation of H₂ pressure (2-10 bar) using 10 mol % KH/C loading, 220 °C, and 10 h). Given the H₂ pressure is reduced by ca. 1 bar due to H₂ consumption, the results show that polystyrene depolymerization can proceed at low H₂ pressure (1-2 bar).

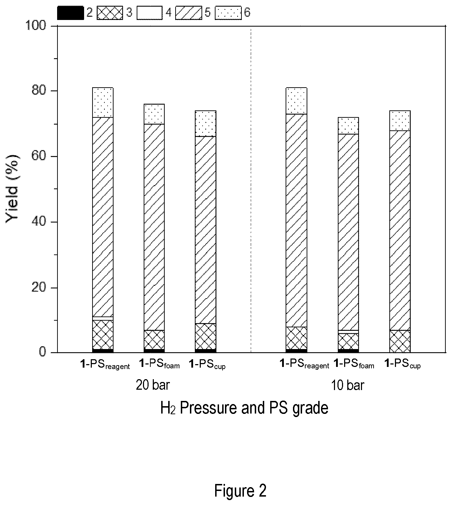

Packaging foam and food-grade cups (denoted as **1**-PS_{foam} and **1**-PS_{cup}, respectively) were tested to assess the applicability of the developed methodology to polystyrene grades beyond the reagent grade. Experiments were conducted with 10 mol % KH/C at 220 °C, 10-20 bar H₂ for 10 h. The combined yields of **3, 5,** and **6** with **1**-PS_{foam} and **1**-PS_{cup} were 70 % and 74 %, respectively, slightly lower than 80 % with **1**-PS_{reagent} (10 bar H₂, Figure 2: Comparison of depolymerization of the reagent grade, package foam, and food-grade polystyrene using 10 mol % KH/C loading, 220 °C, 10 or 20 bar H₂ for 10 h.). These results confirm that consumer-grade polystyrene also succumbs to the developed depolymerization methodology.

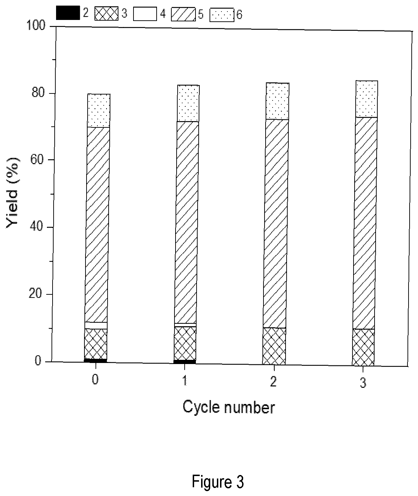

Experiments were conducted to probe the recyclability of the KH/C initiator material in polystyrene depolymerization. Using 10 mol % KH/C loading, 220°C temperature, 30 bar H₂, and 10 h duration, KH/C was recycled 3 times without a notable change in product yield (Figure 3: Recyclability tests of KH/C using 10 mol % KH/C loading, 220 °C, 30 bar H₂, and 10 h.).

To evaluate if styrene-derived copolymers can be depolymerized using our methodology, styrene-butadiene rubber **(11)** was tested. With a 20 mol% KH/C loading under 220 °C, 50 bar H₂ and 10 h duration, reagent grade SBR (3.75 mmol calculated based on the styrene's molecular weight, ca. 30 wt % styrene content, average Mw ca. 140,000 according to GPC) gives 7 % toluene **(3),** 1 % ethylcyclohexane **(4),** 70 % ethylbenzene **(5)** and 8 % cumene (Scheme 1). After removing volatiles in the vacuum, the wax product was obtained as a residue and characterized by ¹H NMR and ¹³C NMR. No aromatic signals were observed, while aliphatic and olefinic signals were observed, consistent with polybutadiene fragments present in the wax. This result suggests that the KH/C methodology can depolymerize the SBR co-polymer by predominantly affecting styrene-derived fragments and retaining polybutadiene fragments of the co-polymer. The scheme below shows the depolymerization of SBR with 20 mol% KH/C loading under 220 °C, 50 bar H₂, and 10 h.

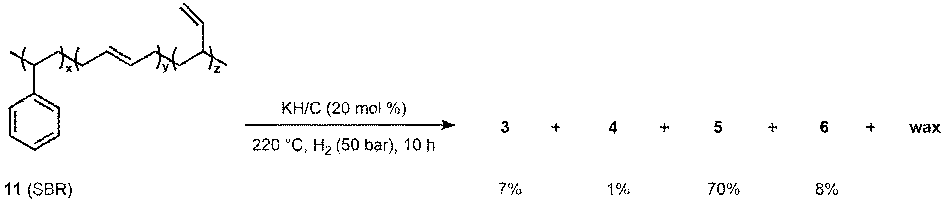

### Methods of in-situ generation of alkali metal hydrides (MH)

With 10 mol % loading of the Stoltz-Grubbs reagent (KOtBu + Et₃SiH, 1:1) under 220 °C and 50 bar H₂ for 20 h, the combined yield of **3, 5** and **6** were 56 % (eq. 1, Scheme 2). The Stoltz-Grubbs reagent has been suggested to produce KH in situ upon heating (Helv. Chim. Acta 2019, 102, e1900235).

With 10 mol % loading of *n*-BuLi (2.5 M in hexane) under the same reaction conditions, the combined yield of **3, 5,** and **6** were 75 % (eq. 2, Scheme 2). *n*-BuLi is known to generate LiH *in situ* by β-elimination upon heating (Z. Anorg. Allg. Chem. 2018, 194-204). Scheme 2 (*In-situ* generation of MH for PS depolymerization with 10 mol % loading of Stoltz-Grubbs reagent or n-BuLi under 220 °C, 50 bar H₂, and 20 h.):

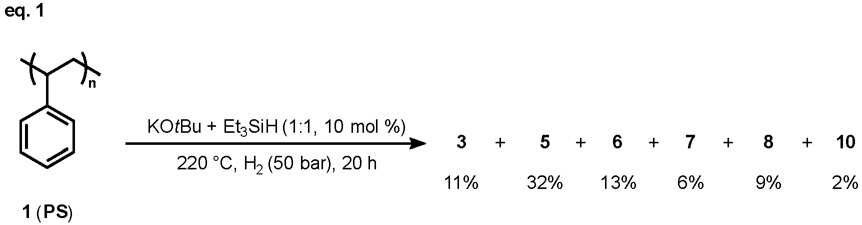

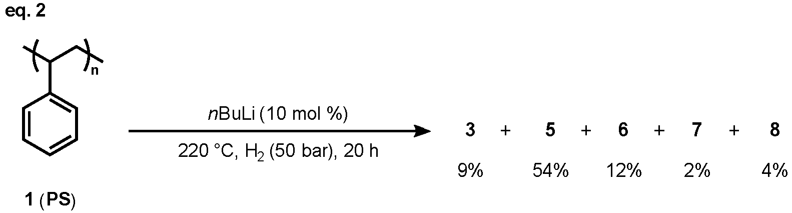

### Vinyl heterocycle polymers

To evaluate if vinyl heterocycle polymers can also be depolymerized using our methodology, poly(4-vinylpyridine) **(12)** was tested. With a 10 mol% KH/C loading under 220 °C, 50 bar H₂ and 10 h duration, reagent grade poly(4-vinylpyridine) (3.75 mmol calculated based on the monomer's molecular weight, 2 % cross-linked with divinylbenzene) gives 9 % 4-methylpyridine **(13),** 17 % 4-ethylpyridine **(14)** and 11 % 4-isopropylpyridine **(15)** (Scheme 3: Depolymerization of poly(4-vinylpyridine) with 10 mol % KH/C loading under 220 °C, 50 bar H₂, and 10 h.):

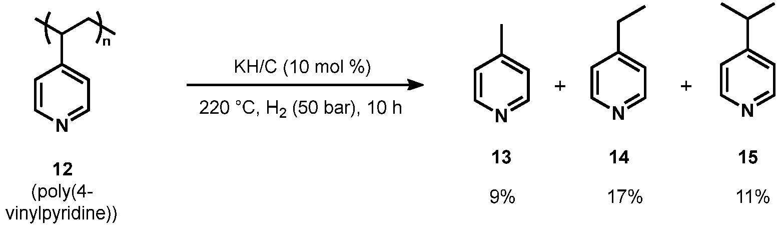

### Styrene acrylonitrile resin (SAN)

With a 1.25 equiv. KH/C loading (100 mg) under 220 °C, 50 bar H₂ and 10 h duration, reagent grade styrene acrylonitrile resin (SAN) (0.6 mmol calculated based on the styrene's molecular weight, ca. 75 wt % styrene content, average Mw ca. 165,000 according to GPC) gives 1 % toluene **(3),** 2 % ethylcyclohexane **(4),** 7 % ethylbenzene **(5)** and 1 % cumene **(6)** ( Scheme 4: Depolymerization of SAN with 1.25 equiv. KH/C loading under 220 °C, 50 bar H₂, and 10 h.).

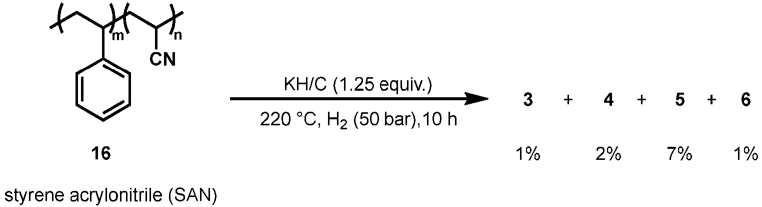

## Claims

1. Method for depolymerizing vinyl-based aromatic or heteroaromatic polymers or copolymers in the absence of a heterogenous nickel catalyst, including the steps of
a) providing a strong electron donor and a vinyl-based aromatic or heteroaromatic polymer or copolymer,
b) reacting the vinyl-based aromatic or heteroaromatic polymer or copolymer with the strong electron donor in the presence of H₂ at a temperature between 130°C and 275°C to obtain platform chemicals.

2. Method according to claim 1, wherein the strong electron donor is selected from the group consisting of a metal hydride, an *in situ* generated alkali metal hydride, alkali naphtalide, alkali diaryl dianion salt, alkalides, and electrides.

3. Method according to any of the preceding claims, wherein the metal hydride, preferably the alkali metal hydride, is used in the presence of a support, preferably selected from the group consisting of carbon, MgO, ZnO, CaO, silica, alumina, zeolites, and mesoporous silica, preferably carbon.

4. Method according to any of the preceding claims, wherein the strong electron donor is a metal hydride, preferably an alkali metal hydride and most preferably an alkali metal hydride selected from the group consisting of potassium hydride, sodium hydride, and lithium hydride.

5. Method according to any of the preceding claims, wherein the alkali metal hydride is potassium hydride, preferably potassium hydride on carbon.

6. Method according to any of claims 1 or 2, wherein the alkali metal hydride is generated *in situ* from potassium alkoxides and silanes, preferably from KOtBu and Et₃SiH,

7. Method according to any of claims 1 or 2, wherein the alkali metal hydride is generated *in situ* from M₁OH and Mg, wherein M₁ is selected from the group consisting of sodium, potassium, rubidium and cesium.

8. Method according to any of the preceding claims, wherein H₂ is added with a pressure of 1 to 50 bar, preferably 5 to 30 bar.

9. Method according to any of the preceding claims, wherein the reaction is conducted in an apolar solvent, preferably decane and dodecane, most preferably dodecane.

10. Method according to any of the preceding claims, wherein the concentration of the strong electron donor is between 1 and 20 mol%, preferably 5 and 15 mol%, more preferably 10 mol%.

11. Method according to any of the preceding claims, wherein the temperature is between 160°C and 250°C, preferably 160°C to 240°C, most preferably 160°C to 220°C.

12. Method according to any of the preceding claims, wherein the reaction occurs over a duration of 1 hour and 12 hours.

13. Method according to any of the preceding claims, wherein the vinyl-based aromatic or heteroaromatic polymers or copolymer is a styrenic polymer.

14. Method according to claim 13 wherein the styrenic polymer is polystyrene, preferably selected from the group consisting of expanded polystyrene (EPS) and extruded polystyrene (XPS).

15. Method according to any of claim 1 to 13, wherein the styrenic polymer is a copolymer, preferably selected from the group consisting of styrene-butadiene Rubber (SBR), acrylonitrile-butadiene-styrene (ABS), styrene-acrylonitrile (SAN), styrene-ethylene-butylene-styrene (SEBS), styrene-isoprene-styrene (SIS), styrene-maleic anhydride (SMA), poly(styrene-co-maleic anhydride), and styrene-ethylene/propylene-styrene (SEPS), preferably styrene-butadiene Rubber (SBR), acrylonitrile-butadiene-styrene (ABS), styrene-acrylonitrile (SAN), styrene-ethylene-butylene-styrene (SEBS), styrene-isoprene-styrene (SIS), and styrene-ethylene/propylene-styrene (SEPS).
